# FASCICULE DE BREVET EUROPEEN

(11) **EP 2 734 490 B1**
(45) Date de publication et mention de la délivrance du brevet: **16.09.2015**
(21) Numéro de dépôt: 12748723.9
(22) Date de dépôt: 09.07.2012
(51) Int. Cl.: C07C 1/24, C07C 11/04, C07C 7/04

(54) **PROCÉDÉ DE DÉSHYDRATATION DE L'ÉTHANOL EN ÉTHYLÈNE A BASSE CONSOMMATION ÉNERGÉTIQUE**
NIEDRIGENERGIEVERBRAUCHSVERFAHREN ZUR DEHYDRIERUNG VON ETHANOL IN ETHYLEN
LOW-ENERGY CONSUMPTION METHOD FOR DEHYDRATING ETHANOL INTO ETHYLENE

(30) Priorité: 21.07.2011 FR 1102274; 08.08.2011 FR 1102482
(43) Date de publication de la demande: 28.05.2014
(73) Titulaire: IFP Energies nouvelles, 92852 Rueil-Malmaison Cedex (FR); Total Research & Technology Feluy, 6520 Seneffe (Feluy) (BE)
(72) Inventeur: COUPARD, Vincent, 69100 Villeurbanne (FR); TOUCHAIS, Natacha, 38200 Vienne (FR); FLEURIER, Stéphanie, 69007 Lyon (FR); GONZALEZ PENAS, Helena, 69007 Lyon (FR); VERMEIREN, Walter, 3530 Houthalen-Helchteren (BE); MINOUX, Delphine, 1400 Nivelles (BE); DE SMEDT, Philip, 9100 Sint-Nillaas (BE); ADAM, Cindy, 5100 Wierde (BE)
(86) Numéro de dépôt international: PCT/FR2012/000279
(87) Numéro de publication internationale: WO 2013/011208

(56) Documents cités:
- WO-A2-2007/134415
- WO-A2-2011/002699
- US-A- 4 396 789

## Description

### Domaine de l'invention

La présente invention se rapporte à un procédé de transformation de l'éthanol en éthylène et en particulier à un procédé de déshydratation de l'éthanol.

### État de la technique antérieure

La réaction de déshydratation de l'éthanol en éthylène est connue et détaillée depuis la fin du XIXème siècle. Il est connu que cette réaction est très endothermique, équilibrée et déplacée vers l'éthylène à haute température. La chute de température correspondant à la conversion totale de l'éthanol pur est de 380°C. Le catalyseur de référence souvent utilisé est un catalyseur monofonctionnel acide. L'alumine gamma est le catalyseur le plus cité. "The Deshydration of Alcohols over Alumina. I:The reaction scheme", H. Knözinger, R. Köhne, Journal of Catalysis (1966), 5, 264-270 est considérée comme la publication de base sur les travaux de déshydratation des alcools dont l'éthanol. Les zéolithes sont aussi utilisées pour cette application, et en particulier la ZSM5 depuis les années 1980, comme par exemple dans "Reactions of ethanol over ZSM-5",S.N. Chaudhuri & al., Journal of Molecular Catalysis 62:289-295 (1990).

Le brevet US 4,232,179 décrit un procédé de déshydratation de l'éthanol en éthylène dans lequel la chaleur nécessaire à la réaction est apportée par l'introduction dans le réacteur, d'un fluide caloporteur en mélange avec la charge. Le fluide caloporteur est soit de la vapeur d'eau provenant d'une source externe, soit un flux externe provenant du procédé, soit le recycle d'une partie de l'effluent du réacteur de déshydratation, c'est à dire l'éthylène produit. L'introduction du mélange de la charge avec ledit fluide caloporteur permet de fournir la chaleur nécessaire au maintien de la température du lit catalytique à un niveau compatible aux niveaux de conversions désirés. Dans le cas où le fluide caloporteur est l'effluent du réacteur de déshydratation, un compresseur de recyclage dudit effluent est nécessaire. Cependant, le recyclage de l'éthylène produit par la réaction est un inconvénient car l'introduction de l'éthylène modifie l'équilibre de la réaction de déshydratation. De plus, l'éthylène participe aux réactions secondaires d'oligomérisation, de transfert d'hydrogène et de disproportionation des oléfines qui sont des réactions d'ordre supérieur à 0 par rapport à leur réactif. L'augmentation de la concentration en éthylène dès le début de la réaction multiplie la formation de produits secondaires. La perte en éthylène est donc plus importante, ce qui traduit une baisse de la sélectivité.

La demande de brevet WO 2007/134415 A2 décrit un procédé de déshydratation de l'éthanol en éthylène amélioré par rapport à celui du brevet US 4 232 179 permettant un coût d'investissement réduit, grâce à un nombre réduit d'équipements et un coût opérationnel réduit, grâce à la non utilisation de vapeur d'eau externe au procédé. Dans ce procédé, au moins une partie de l'effluent du réacteur de déshydratation (mélange d'éthylène produit et de vapeur d'eau) et de la vapeur d'eau surchauffée obtenue à partir de l'eau produite par la déshydratation de l'éthanol et condensée dans le réacteur, sont utilisés comme fluide caloporteur et entrent dans le réacteur de déshydratation en mélange avec l'éthanol. Par ailleurs, ladite demande de brevet est muette sur la condition de pression à respecter entre la charge éthanol et l'effluent dans le but de maximiser l'échange de chaleur. WO2011/002699 divulgue un procédé de déshydratation d'une charge éthanol en éthylène comprenant la vaporisation d'un mélange d'éthanol et d'eau et la réaction de ce mélange dans un réacteur adiabatique.

Le brevet US 4,396,789 décrit également un procédé de déshydratation de l'éthanol en éthylène dans lequel l'éthanol et la vapeur d'eau agissant comme fluide caloporteur sont introduits dans le premier réacteur à une température comprise entre 400 et 520°C et à une pression élevée comprise entre 20 et 40 atm, de sorte que l'effluent produit par la réaction de déshydratation est soutiré du dernier réacteur à une pression au moins supérieure à 18 atm, ledit produit de réaction, c'est à dire l'éthylène, après refroidissement, pouvant subir l'étape de distillation cryogénique finale sans étape de compression intermédiaire. Ledit procédé se caractérise également par un échange de chaleur entre ledit produit de la réaction de déshydratation et la charge introduite dans le premier réacteur, ledit produit de réaction étant utilisé pour vaporiser la charge entrant dans le premier réacteur. L'éthanol non réagi, au moins une partie de l'eau formée au cours des réactions du procédé et l'eau ajoutée pour le lavage final des gaz sont recyclés pour assurer la conversion complète de l'éthanol.

Un objectif de l'invention est de fournir un procédé de déshydratation de l'éthanol en éthylène dans lequel la charge est introduite dans l'étape a) de vaporisation de la charge à une pression faible, inférieure à la pression de réaction, de sorte que ledit procédé ne nécessite aucun fluide caloporteur externe au procédé. En particulier, la charge est introduite dans l'étape a) de vaporisation de la charge à une pression inférieure à la pression de l'effluent en sortie du dernier réacteur de manière à maximiser l'échange de chaleur entre la charge et l'effluent issu du dernier réacteur, c'est à dire à échanger la totalité de l'enthalpie de vaporisation de la charge et l'enthalpie de condensation dudit effluent.

Un autre objectif de l'invention est de fournir un procédé de déshydratation de l'éthanol en éthylène de pureté élevée, ledit procédé permettant d'augmenter la sélectivité en éthylène avec une consommation spécifique par tonne d'éthylène produit significativement abaissée par rapport aux procédés de l'art antérieur.

### Résumé et intérêt de l'invention

L'invention décrit un procédé de déshydratation d'une charge éthanol en éthylène comprenant :
a) la vaporisation de ladite charge éthanol en mélange avec au moins une partie du flux d'eau purifié recyclé selon l'étape f) dans un échangeur grâce à un échange de chaleur avec l'effluent issu du dernier réacteur, ladite charge éthanol en mélange avec au moins une partie dudit flux d'eau purifié étant introduite dans ladite étape de vaporisation à une pression comprise entre 0,1 et 0,4 MPa,
b) la compression de ladite charge vaporisée dans un compresseur,
c) l'introduction de ladite charge vaporisée et comprimée, à une température d'entrée comprise entre 350 et 500°C et à une pression d'entrée comprise entre 0,2 et 1,3 MPa, dans au moins un réacteur adiabatique contenant au moins un catalyseur de déshydratation et dans lequel la réaction de déshydratation a lieu,
d) la séparation de l'effluent issu du dernier réacteur adiabatique de l'étape c) en un effluent comprenant de l'éthylène à une pression inférieure à 1 MPa et un effluent comprenant de l'eau,
e) la purification d'au moins une partie de l'effluent comprenant de l'eau issu de l'étape d) et la séparation d'au moins un flux d'eau purifiée et d'au moins un flux d'éthanol non converti,
f) le recyclage d'au moins une partie du flux d'eau purifiée issu de l'étape e) en amont de l'étape a).

La présente invention présente l'avantage par rapport aux procédés de l'art antérieur de maximiser l'échange de chaleur entre la charge et l'effluent issu du dernier réacteur, c'est à dire d'échanger la totalité de l'enthalpie de vaporisation de la charge et la majeure partie de l'enthalpie de condensation dudit effluent grâce à l'introduction de la charge dans l'étape a) de vaporisation à une pression inférieure à la pression de l'effluent en sortie du dernier réacteur.

### Description de l'invention

La charge éthanol traitée dans le procédé selon l'invention est éventuellement obtenue par un procédé de synthèse d'alcool à partir de ressources fossiles telles que par exemple à partir du charbon, du gaz naturel ou des déchets carbonés.

Ladite charge peut également avantageusement provenir de ressources non fossiles. De préférence, la charge éthanol traitée dans le procédé selon l'invention est une charge éthanol produite à partir de source renouvelable issue de la biomasse et est souvent appelée "bioéthanol". Ladite charge éthanol est une charge produite par voie biologique, de préférence par fermentation de sucres issus par exemple des cultures de plantes sucrières comme la canne à sucre (saccharose, glucose, fructose, et sucrose), des betteraves, ou encore des plantes amylacées (amidon) ou de la biomasse lignocellulosique ou de cellulose hydrolysée (glucose majoritaire et xylose, galactose), contenant des quantités variables d'eau.

Ladite charge est avantageusement obtenue par fermentation à partir de trois sources : 1) le sucrose de canne ou de betterave, 2) l'amidon présent dans les céréales et les tubercules et 3) la cellulose et l'hémicellulose présentes dans le bois, les herbes et autres biomasses lignocellulosiques, l'amidon, la cellulose et l'hémicellulose devant être hydrolysées en sucres avant de subir une étape de fermentation.

Pour une description plus complète des procédés fermentaires classiques, on peut se reporter a l'ouvrage 'Les Biocarburants, Etat des lieux, perspectives et enjeux du développement, Daniel Ballerini, Editions Technip'.

Ladite charge peut avantageusement également être obtenue par fermentation de gaz de synthèse. Ladite charge peut également avantageusement également être obtenue par hydrogénation des acides ou esters correspondants. Dans ce cas, l'acide acétique ou les esters acétiques sont avantageusement hydrogénés à l'aide d'hydrogène en éthanol. L'acide acétique peut avantageusement être obtenu par carbonylation du méthanol ou par fermentation des carbohydrates. De préférence, la charge éthanol traitée dans le procédé selon l'invention est une charge éthanol produite à partir de source renouvelable issue de la biomasse.

La charge éthanol utilisée selon l'invention est avantageusement une charge éthanol hydratée concentrée. On entend par charge éthanol hydratée concentrée une charge éthanol comprenant un pourcentage massique en éthanol supérieur ou égal à 35% poids. De préférence, ladite charge éthanol concentrée comprend un pourcentage massique en éthanol compris entre 35 et 99,9% poids. De manière préférée, ladite charge éthanol concentrée comprend un pourcentage massique en éthanol compris entre 35 et 96% poids. Ladite charge éthanol concentrée comprend également avantageusement, en plus de l'eau, une teneur en alcools autres que l'éthanol, tels que par exemple le méthanol, le butanol et/ou l'isopentanol inférieure à 10% poids, et de préférence inférieure à 5% poids, une teneur en composés oxygénés autres que les alcools tels que par exemple les éthers, les acides, les cétones, les aldéhydes et/ou les esters avantageusement inférieure à 1% poids et une teneur en azote et en soufre, organique et minéral, avantageusement inférieure à 0,5% poids, les pourcentages poids étant exprimés par rapport à la masse totale de ladite charge

La charge éthanol utilisée selon l'invention subit avantageusement une étape de prétraitement préalablement à l'étape a) de vaporisation de ladite charge. Ladite étape de prétraitement permet d'éliminer les impuretés contenues dans ladite charge de manière à limiter la désactivation du catalyseur de déshydratation placé en aval, et en particulier les composés contenant de l'azote et les composés contenant du soufre. Les composés oxygénés présents dans ladite charge ne sont pas substantiellement éliminés.

Ladite étape de prétraitement est avantageusement mise en oeuvre par des moyens connus de l'homme du métier, tels que par exemple l'utilisation d'au moins une résine, par l'adsorption des impuretés sur des solides de préférence à une température comprise entre 20 et 60°C, par un enchainement comprenant une première étape d'hydrogénolyse opérant à une température comprise entre 20 et 80°C, suivie d'une étape de captation sur solide acide à une température comprise entre 20 et 80°C et/ou par la distillation. Dans le cas de l'utilisation d'au moins une résine, ladite résine est de préférence acide et est utilisée à température élevée comprise entre 70 et 200°C. Ladite résine peut éventuellement être précédée d'une résine basique.

Dans le cas où l'étape de prétraitement est mise en oeuvre par l'adsorption des impuretés sur des solides, lesdits solides sont avantageusement choisis parmi les tamis moléculaires, le charbon actif, l'alumine et les zéolithes.

Ladite étape de prétraitement de la charge éthanol permet de produire une coupe purifiée d'éthanol dans laquelle les impuretés organiques ont été éliminées, afin d'obtenir une charge purifiée répondant au niveau d'impuretés compatibles avec le catalyseur de déshydratation.

### Étape a)

Selon l'invention, le procédé de déshydratation comprend une étape a) de vaporisation de la dite charge éthanol, éventuellement prétraitée, en mélange avec au moins une partie du flux d'eau purifié recyclé selon l'étape f), dans un échangeur grâce à un échange de chaleur avec l'effluent issu du dernier réacteur adiabatique, ladite charge éthanol, en mélange avec au moins une partie du flux d'eau purifié recyclé selon l'étape f), étant introduite dans ladite étape de vaporisation à une pression comprise entre 0,1 et 0,4 MPa.

De préférence, au moins un flux d'éthanol non réagi issu de l'étape e) de purification de l'effluent comprenant de l'eau est également introduit, en mélange avec ladite charge éthanol, éventuellement prétraitée, et en mélange avec au moins une partie du flux d'eau purifié recyclé selon l'étape f), dans l'échangeur de l'étape a) de vaporisation.

De préférence, ladite charge éthanol est mélangée avec au moins une partie du flux d'eau purifié recyclé selon l'étape f) et avec au moins un flux d'éthanol non réagi issu de l'étape e) de purification de l'effluent comprenant de l'eau, après l'étape de prétraitement de ladite charge éthanol.

De préférence, ladite charge éthanol, en mélange avec au moins une partie du flux d'eau purifié recyclé selon l'étape f) et éventuellement en mélange avec au moins un flux d'éthanol non réagi issu de l'étape e), est introduite dans ladite étape a) de vaporisation à une pression inférieure à la pression de l'effluent en sortie du dernier réacteur.

Un critère essentiel de la présente invention est l'ajustement de la pression en amont de l'étape a) de vaporisation de ladite charge éthanol en mélange avec au moins une partie du flux d'eau purifié recyclé selon l'étape f) et éventuellement en mélange avec au moins un flux d'éthanol non réagi issu de l'étape e), de manière à maximiser l'échange de chaleur entre le mélange de ladite charge et des différents flux et l'effluent issu du dernier réacteur adiabatique. L'introduction de ladite charge éthanol en mélange avec au moins une partie du flux d'eau purifié recyclé selon l'étape f) et éventuellement en mélange avec un flux d'éthanol non réagi issu de l'étape e), dans l'étape a) de vaporisation à ce niveau de pression spécifique compris entre 0,1 et 0,4 MPa, inférieure à la pression de l'effluent en sortie du dernier réacteur, permet de bénéficier d'une température de vaporisation du mélange de charge inférieure à la température de condensation de l'effluent issu du dernier réacteur adiabatique. Ainsi, la majeure partie de la chaleur latente de la phase aqueuse de l'effluent issu du dernier réacteur adiabatique est récupérée pour vaporiser ledit mélange de ladite charge et des différents flux, sans apport de chaleur externe. La totalité de l'enthalpie de vaporisation dudit mélange de ladite charge et des différents flux est donc échangée avec l'enthalpie de condensation dudit effluent.

La pression de ladite charge éthanol, en mélange avec au moins une partie du flux d'eau purifié recyclé selon l'étape f) et éventuellement en mélange avec au moins un flux d'éthanol non réagi issu de l'étape e), au niveau de sa vaporisation est avantageusement choisie de façon à ce que l'écart de température entre l'effluent issu du dernier réacteur adiabatique qui se condense et ledit mélange de charge qui s'évapore est toujours au moins supérieure à 2°C, et de préférence au moins supérieur à 3°C.

### Étape b)

Selon l'invention, ladite charge éthanol en mélange avec au moins une partie du flux d'eau purifié recyclé selon l'étape f) et éventuellement en mélange avec au moins un flux d'éthanol non réagi issu de l'étape e), vaporisée, subit une compression dans un compresseur. L'étape b) de compression est avantageusement mise en oeuvre dans tout type de compresseur connus de l'homme du métier. En particulier, L'étape b) de compression est avantageusement mise en oeuvre dans un compresseur de type compresseur radial à multiplicateur intégré ou dans un compresseur comprenant une ou plusieurs soufflantes avec une roue radiale mises en série sans refroidissement intermédiaire. L'étape b) de compression de ladite charge éthanol en mélange avec au moins une partie du flux d'eau purifié recyclé selon l'étape f) et éventuellement en mélange avec au moins un flux d'éthanol non réagi issu de l'étape e), vaporisée, permet d'éviter l'apport de fluide caloporteur externe au procédé pour assurer la vaporisation dudit mélange de ladite charge et des différents flux. Ainsi, Seuls les flux issus du procédé sont utilisés. L'étape b) de compression permet donc de réaliser une pompe à chaleur intégrée audit procédé, utilisant les flux issus du procédé, et ne faisant pas intervenir de fluide caloporteur externe.

La combinaison des conditions opératoires spécifiques de l'étape a) et l'étape b) permet de récupérer la majeure partie de la chaleur latente de la phase aqueuse de l'effluent issu du dernier réacteur adiabatique pour vaporiser la charge éthanol en mélange avec au moins une partie du flux d'eau purifié recyclé selon l'étape f) et éventuellement en mélange avec au moins un flux d'éthanol non réagi issu de l'étape e), sans apport de chaleur externe, c'est à dire d'échanger la totalité de l'enthalpie de vaporisation dudit mélange de ladite charge et des différents flux et la majeure partie de l'enthalpie de condensation dudit effluent.

La pression de ladite charge éthanol en mélange avec au moins une partie du flux d'eau purifié recyclé selon l'étape f) et éventuellement en mélange avec au moins un flux d'éthanol non réagi issu de l'étape e), vaporisée à l'issue de l'étape b) de compression, est avantageusement comprise entre 0,2 et 1,3 MPa. La pression de sortie dudit mélange de ladite charge et des différents flux est suffisante pour réaliser la condition de température nécessaire à l'échange de l'étape a) : dans l'étape a), la température de vaporisation dudit mélange de ladite charge et des différents flux doit être inférieure à la température de condensation de l'effluent issu du dernier réacteur.

Ladite charge éthanol en mélange avec au moins une partie du flux d'eau purifié recyclé selon l'étape f) et éventuellement en mélange avec au moins un flux d'éthanol non réagi issu de l'étape e), vaporisée et comprimée, issue de l'étape b) de compression est éventuellement chauffée dans un échangeur de type monophasique gaz, grâce à un échange de chaleur avec l'effluent issu du dernier réacteur adiabatique de l'étape c). Dans ledit échangeur de type monophasique gaz, ledit mélange de ladite charge et des différents flux, vaporisé et comprimé est surchauffé et l'effluent issu, à l'état gazeux, du dernier réacteur adiabatique de l'étape c) est "désurchauffé" sans être condensé.

Ledit mélange de ladite charge et des différents flux est avantageusement surchauffée à une température comprise entre 250 et 375°C et de préférence comprise entre 280 et 360°C. A l'issu dudit échangeur de type monophasique gaz, l'effluent issu, à l'état gazeux, du dernier réacteur adiabatique de l'étape c) présente avantageusement une température comprise entre 180 et 220°C.

Ainsi, l'utilisation des différents échangeurs, de type monophasique gaz et vaporiseur gaz/liquide, et la vaporisation, à une pression inférieure à la pression de l'effluent en sortie du dernier réacteur, de ladite charge éthanol en mélange avec au moins une partie du flux d'eau purifié recyclé selon l'étape f) et éventuellement en mélange avec au moins un flux d'éthanol non réagi issu de l'étape e), permet la condensation d'au moins 80% des vapeurs d'eau présentes dans l'effluent issu du dernier réacteur.

Ledit mélange de charge, vaporisé, comprimé et éventuellement chauffé dans ledit échangeur de type monophasique gaz est ensuite avantageusement introduit dans un four de manière à l'amener à une température d'entrée dans au moins un réacteur adiabatique compatible avec la température de la réaction de déshydratation.

### Étape c)

Selon l'invention, ladite charge éthanol en mélange avec au moins une partie du flux d'eau purifié recyclé selon l'étape f) et éventuellement en mélange avec au moins un flux d'éthanol non réagi issu de l'étape e), vaporisée et comprimée, et éventuellement chauffée, est introduite à une température d'entrée comprise entre 350 et 500°C et à une pression d'entrée comprise entre 0,2 et 1,3 MPa dans au moins un réacteur adiabatique contenant au moins un lit fixe de catalyseur de déshydratation et dans lequel la réaction de déshydratation a lieu.

L'effluent issu du dernier réacteur adiabatique de l'étape c) présente avantageusement, en sortie du dernier réacteur adiabatique de l'étape c) une température comprise entre 270 et 420°C et de préférence comprise entre 300 et 410°C.

L'effluent issu du dernier réacteur adiabatique de l'étape c) présente avantageusement, en sortie du dernier réacteur adiabatique de l'étape c) une pression comprise entre 0,1 et 1,1 MPa.

L'étape c) dans laquelle la réaction de déshydratation a lieu est avantageusement réalisée dans un ou deux réacteurs.

Dans le cas où l'étape c) est mise en oeuvre dans un réacteur adiabatique, ladite charge éthanol en mélange avec au moins une partie du flux d'eau purifié recyclé selon l'étape f) et éventuellement en mélange avec au moins un flux d'éthanol non réagi issu de l'étape e), vaporisée et comprimée, et éventuellement chauffée, est avantageusement introduite dans ledit réacteur à une température d'entrée comprise entre 400 et 500°C et à une pression d'entrée comprise entre 0,2 et 1,2 MPa. L'effluent issu dudit réacteur adiabatique présente avantageusement une température comprise entre 300 et 400°C et une pression de sortie avantageusement comprise entre 0,1 et 1,1 MPa.

Dans le cas où l'étape c) est mise en oeuvre dans deux réacteurs adiabatiques, ladite charge éthanol en mélange avec au moins une partie du flux d'eau purifié recyclé selon l'étape f) et éventuellement en mélange avec au moins un flux d'éthanol non réagi issu de l'étape e), vaporisée et comprimée, et éventuellement chauffée, est avantageusement introduite dans le premier réacteur à une température d'entrée comprise entre 350 et 450°C et de préférence à une température comprise entre 370 et 420°C, et à une pression d'entrée comprise entre 0,3 et 1,3 MPa et de préférence comprise entre 0,4 et 0,8 MPa.

L'effluent issu du premier réacteur adiabatique sort avantageusement dudit premier réacteur à une température comprise entre 290 et 390°C et à une pression comprise entre 0,2 et 1,2 MPa.

Ledit effluent est ensuite avantageusement introduit dans un four de manière à ce que la température d'entrée dudit effluent dans le deuxième réacteur adiabatique soit comprise entre 350 et 450 °C et de préférence entre 370 et 430°C. Ledit effluent présente une pression d'entrée dans ledit deuxième réacteur avantageusement comprise entre 0,2 et 1,2 MPa et de préférence entre 0,3 et 0,7 MPa. L'effluent issu du deuxième réacteur adiabatique sort dudit deuxième réacteur adiabatique à une température avantageusement comprise entre 310 et 410°C. La pression de sortie dudit effluent issu du deuxième réacteur adiabatique est avantageusement comprise entre 0,1 et 1,1 MPa.

La température d'entrée du ou des réacteurs peut avantageusement être graduellement augmentée pour éviter la désactivation du catalyseur de déshydratation.

La réaction de déshydratation qui a lieu dans au moins un réacteur adiabatique de l'étape c) du procédé selon l'invention opère avantageusement à une vitesse pondérale horaire comprise entre 0,1 et 20 h-1 et de préférence entre 0,5 et 15 h-1. La vitesse pondérale horaire est définie comme étant le rapport du débit massique de la charge éthanol pure sur la masse de catalyseur.

Le catalyseur de déshydratation utilisé dans l'étape c) est un catalyseur connu de l'homme du métier. Ledit catalyseur est de préférence un catalyseur acide amorphe ou un catalyseur acide zéolithique.

Dans le cas où le catalyseur de déshydratation utilisé dans l'étape c) est un catalyseur zéolithique, ledit catalyseur comprend au moins une zéolithe choisie parmi les zéolithes ayant au moins des ouvertures de pores contenant 8, 10 ou 12 atomes d'oxygènes (8 MR, 10MR ou 12 MR). Il est connu en effet de définir la taille des pores des zéolithes par le nombre d'atomes d'oxygène formant la section annulaire des canaux des zéolithes, appelés "member ring" ou MR en anglais. De manière préférée, ledit catalyseur de déshydratation zéolithique comprend au moins une zéolithe présentant un type structural choisi parmi les types structuraux MFI, MEL, FAU, MOR, FER, SAPO, TON, CHA, EUO et BEA. De préférence, ledit catalyseur de déshydratation zéolithique comprend une zéolithe de type structural MFI et de manière préférée une zéolithe ZSM-5.

La zéolithe mise en oeuvre dans le catalyseur de déshydratation utilisé dans l'étape c) du procédé selon l'invention peut avantageusement être modifiée par désalumination ou désilication selon toute méthode de désalumination ou désilication connue de l'homme du métier.

La zéolithe mise en oeuvre dans le catalyseur de déshydratation utilisé dans l'étape c) du procédé selon l'invention ou le catalyseur final peut avantageusement être modifié par un agent de nature à atténuer son acidité totale et à améliorer ses propriétés de résistance hydrothermales. De préférence, ladite zéolithe ou ledit catalyseur comprend avantageusement du phosphore, de préférence ajouté sous forme H₃PO₄ suivi d'un traitement à la vapeur après neutralisation de l'excès d'acide par un précurseur basique tels que par exemple le sodium Na ou le calcium ca. De manière préférée, ladite zéolithe comprend une teneur en phosphore comprise entre 2,5 et 4,5% poids par rapport à la masse totale du catalyseur.

De préférence, le catalyseur de déshydratation utilisé dans l'étape c) du procédé selon l'invention est le catalyseur décrit dans les demandes de brevet WO/2009/098262, WO/2009/098267, WO/2009/098268 ou WO/2009/098269.

Dans le cas où le catalyseur de déshydratation utilisé dans l'étape c) est un catalyseur acide amorphe, ledit catalyseur comprend au moins un oxyde réfractaire poreux choisi parmi l'alumine, l'alumine activée par un dépôt d'acide minéral et la silice alumine.

Ledit catalyseur de déshydratation amorphe ou zéolithique utilisé dans l'étape c) du procédé selon l'invention peut avantageusement également comprendre au moins une matrice de type oxyde également appelé liant. On entend par matrice selon l'invention, une matrice amorphe ou mal cristallisée. Ladite matrice est avantageusement choisie parmi les éléments du groupe formé par les argiles (telles que par exemple parmi les argiles naturelles telles que le kaolin ou la bentonite), la magnésie, les alumines, les silices, les silice-alumines, les aluminates, l'oxyde de titane, l'oxyde de bore, la zircone, les phosphates d'aluminium, les phosphates de titane, les phosphates de zirconium, et le charbon. De préférence ladite matrice est choisie parmi les éléments du groupe formé par les alumines, les silices et les argiles.

Ledit catalyseur de déshydratation utilisé dans l'étape c) du procédé selon l'invention est avantageusement mis en forme sous la forme de grains de différentes formes et dimensions. Il est avantageusement utilisé sous la forme d'extrudés cylindriques ou polylobés tels que bilobés, trilobés, polylobés de forme droite ou torsadée, mais peut éventuellement être fabriqué et employé sous la forme de poudre concassée, de tablettes, d'anneaux, de billes, de roues, de sphères. De préférence, ledit catalyseur est sous forme d'extrudés.

Ledit catalyseur de déshydratation utilisé dans l'étape c) du procédé selon l'invention est avantageusement mis en oeuvre dans au moins un réacteur, en lit fixe ou en lit mobile.

Dans l'étape c) du procédé selon l'invention, les catalyseurs utilisés et les conditions opératoires sont choisis de manière à maximiser la production d'éthylène. La réaction globale de déshydratation mise en oeuvre dans l'étape c) du procédé selon l'invention est la suivante :

2 C₂H₅OH → 2CH₂=CH₂ + 2H₂O

La conversion de la charge éthanol dans l'étape c) est avantageusement supérieure à 90%, de préférence 95% et de manière préférée supérieure à 99%.

La conversion de la charge éthanol est définie, en pourcentage, par la formule suivante : [1 - (masse horaire d'éthanol en sortie/masse horaire d'éthanol en entrée)]x100.

La masse horaire d'éthanol en entrée et en sortie est mesurée de manière classique par chromatographie en phase gazeuse de la phase aqueuse.

L'étape c) dans laquelle la réaction de déshydratation a lieu est avantageusement réalisée dans un ou deux réacteurs. Un réacteur préféré est un réacteur radial fonctionnant en mode ascendant ou descendant. Lors de l'étape c) du procédé selon l'invention, la transformation de la charge s'accompagne de la désactivation du catalyseur de déshydratation par cokage et/ou par adsorption de composés inhibiteurs. Le catalyseur de déshydratation doit donc subir périodiquement une étape de régénération. De préférence, le réacteur est utilisé dans un mode de régénération alterné, aussi appelé réacteur swing, afin d'alterner les phases de réaction et de régénération dudit catalyseur de déshydratation. L'objectif de ce traitement de régénération est de brûler les dépôts organiques ainsi que les espèces contenant de l'azote et du soufre, contenues à la surface et au sein dudit catalyseur de déshydratation.

La régénération du catalyseur de déshydratation utilisé dans ladite étape c) est avantageusement réalisée par oxydation du coke et des composés inhibiteurs sous flux d'air ou sous un mélange air/azote, par exemple en utilisant une recirculation de l'air de combustion avec ou sans eau afin de diluer l'oxygène et maîtriser l'exothermie de régénération. Dans ce cas, on peut avantageusement ajuster la teneur en oxygène en entrée du réacteur par un appoint d'air. La régénération a lieu à pression entre la pression atmosphérique (0 bar relatif) et la pression de réaction. La température de régénération est avantageusement choisie entre 400 et 600°C ; elle peut avantageusement varier en cours de régénération. La fin de la régénération est détectée quand il n'y a plus de consommation d'oxygène, signe d'une combustion totale du coke.

De préférence, l'effluent issu du dernier réacteur adiabatique de l'étape c) n'est pas recyclé en amont de l'étape c), dans au moins un réacteur adiabatique.

L'effluent issu du dernier réacteur adiabatique de l'étape c) est éventuellement envoyé dans un échangeur de type monophasique gaz dans lequel il est "désurchauffé" sans être condensé par échange de chaleur avec la charge vaporisée et comprimée issue de l'étape b), qui elle est réchauffée. Ledit effluent "désurchauffé" est ensuite avantageusement envoyé dans un deuxième échangeur de type gaz/liquide dans lequel il est condensé partiellement par un échange de chaleur servant à vaporiser la charge.

### Étape d)

Selon l'invention, l'effluent issu du dernier réacteur adiabatique de l'étape c) subit une étape de séparation d) en un effluent comprenant de l'éthylène à une pression inférieure à 1 MPa et un effluent comprenant de l'eau.

L'étape d) de séparation dudit effluent issu du dernier réacteur adiabatique de l'étape c) peut avantageusement être mise en oeuvre par toute méthode connue de l'homme du métier telle que par exemple par une zone de séparation gaz/liquide, et de préférence une colonne de séparation gaz/liquide.

L'effluent comprenant de l'éthylène à une pression inférieure à 1 MPa subit ensuite avantageusement une compression. Ladite compression permet de remonter la pression dudit effluent à une pression avantageusement comprise entre 2 et 4 MPa nécessaire à sa purification finale.

De préférence, l'effluent comprenant de l'éthylène séparé à l'issu de l'étape d) n'est pas recyclé dans au moins un réacteur adiabatique de l'étape c). Le non recyclage de l'éthylène séparé à l'issu de l'étape d) dans au moins un réacteur adiabatique de l'étape c) n'altère pas la sélectivité en éthylène du procédé selon l'invention.

Au moins une partie de l'effluent comprenant de l'eau issu de l'étape d) est éventuellement recyclée dans l'étape d) de séparation. Dans le cas où au moins une partie de l'effluent comprenant de l'eau est recyclée, ladite partie de l'effluent comprenant de l'eau est avantageusement refroidie à l'aide d'un fluide froid ou d'un fluide issu du procédé et est de préférence purifiée selon les méthodes connues de purification décrites ci-dessous.

### Étape e)

Selon l'invention, au moins une partie de l'effluent comprenant de l'eau issu de l'étape d) de séparation subit une étape e) de purification. L'étape e) de purification peut avantageusement être mise en oeuvre par toute méthode de purification connue de l'homme du métier. A titre d'exemple, l'étape e) de purification peut avantageusement être mise en oeuvre par l'utilisation de résines échangeuses d'ion, de tamis moléculaires, de membranes, par ajout d'agents chimiques pour ajuster le pH, tels que par exemple la soude ou les amines et par l'ajout d'agent chimiques pour stabiliser les produits, tels que par exemple les inhibiteurs de polymérisation choisis parmi les bisuflites et les tensio-actifs.

Au moins un flux d'eau purifiée et au moins un flux d'éthanol non converti sont ensuite séparés. La séparation peut avantageusement être mise en oeuvre par toute méthode de séparation connue de l'homme du métier. A titre d'exemple, la séparation peut avantageusement être mise en oeuvre par distillation, l'utilisation de tamis moléculaires, de membranes, strippage à la vapeur ou a la chaleur ou par absorption au solvant tels que par exemple les solvants glycolés.

Un flux contenant les gaz légers, de préférence l'acétaldéhyde et le méthanol, peut avantageusement également être séparé.

L'utilisation du flux d'eau purifiée issue de l'étape e) permet de séparer la grande majorité de l'éthylène de l'eau avant son recyclage. On dissocie ainsi dans le procédé selon l'invention l'éthylène du diluant, ce qui permet l'utilisation d'un diluant réactionnel thermique inerte pour le procédé. Ceci permet également une récupération énergétique améliorée, sans dégrader le rendement et la sélectivité en éthylène final.

### Étape f)

Selon l'invention au moins une partie du flux d'eau purifié issu de l'étape e) est recyclé en amont de l'étape a). Au moins une partie du flux d'eau purifiée issu de l'étape e) est mélangé à la charge éthanol éventuellement prétraitée et éventuellement en mélange avec au moins un flux d'éthanol non réagi issu de l'étape e), en amont de l'étape a) de vaporisation de ladite charge.

Le flux d'eau purifié issu de l'étape e) joue le rôle de diluant réactionnel thermique.

La dilution de ladite charge éthanol par ajout d'au moins une partie du flux d'eau purifié issu de l'étape e) est réalisée dans un rapport massique diluant sur charge, avantageusement compris entre 1 et 4 dans le but d'abaisser les pressions partielles d'éthanol dans le ou les réacteurs et de rendre le procédé plus sélectif en éthylène.

Au moins une partie dudit flux d'éthanol non réagi issu de l'étape e) de purification de l'effluent comprenant de l'eau est avantageusement recyclé et mélangé, en amont de l'étape a) de vaporisation, à la charge éthanol éventuellement prétraitée, et mélangé avec au moins une partie du flux d'eau purifié recyclé selon l'étape f) .

### Description des figures

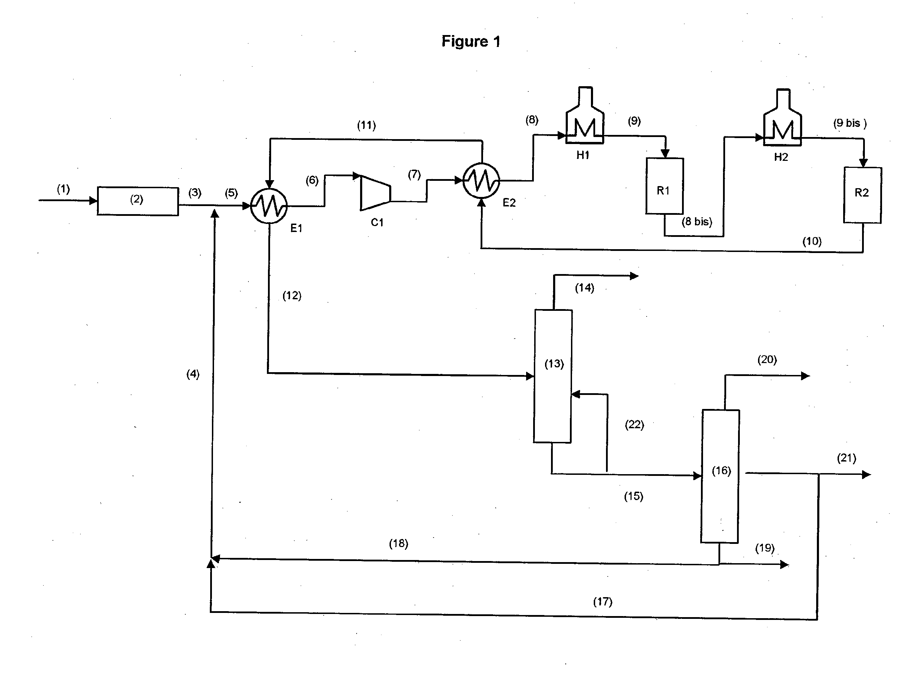

La figure 1 représente schématiquement le procédé de déshydratation de l'éthanol dans le cas de la déshydratation d'une charge éthanol concentrée avec un recyclage d'au moins une partie de l'eau purifiée lors de l'étape f) du procédé.

La charge éthanol est introduite dans une zone de prétraitement (2) via la conduite (1). La charge éthanol prétraitée (3) est ensuite mélangée dans la conduite (5) avec une partie du flux d'eau purifié issu de la zone de purification (16) qui est recyclé en diluant réactionnel via les conduites (18) et (4). La charge éthanol est aussi mélangée avec une partie du flux d'éthanol non réagi issu de la zone de purification (16), via la conduite (17) puis (4). La charge éthanol prétraitée en mélange avec une partie du flux d'eau purifié recyclé et une partie du flux d'éthanol non réagi est introduite via la conduite (5) à une pression comprise entre 0,1 et 0,4 MPa, dans un échangeur gaz/liquide E1 dans lequel ledit mélange subit un échange de chaleur avec l'effluent issu du dernier réacteur adiabatique R2 qui pénètre dans l'échangeur via la conduite (11). La chaleur latente ou enthalpie de condensation de l'effluent issu du dernier réacteur adiabatique R2 est utilisée pour vaporiser la charge éthanol en mélange avec le flux d'eau purifié recyclé et un flux d'éthanol non réagi, sans apport de chaleur externe.

La charge éthanol en mélange avec le flux d'eau purifié recyclé et un flux d'éthanol non réagi, vaporisée, est ensuite envoyée via la conduite (6) dans un compresseur C1.

Ledit mélange de la charge et des deux flux, vaporisé et comprimé est ensuite envoyé via la conduite (7) dans un échangeur E2 de type monophasique gaz, dans lequel ledit mélange est chauffé grâce à un échange de chaleur avec l'effluent issu du dernier réacteur adiabatique R2 qui est introduit dans E2 via la conduite (10). Dans ledit échangeur de type monophasique gaz, ladite charge vaporisée et comprimée est surchauffée et l'effluent issu, à l'état gazeux, du dernier réacteur adiabatique R2 est "désurchauffé" sans être condensé.

Ledit mélange de la charge et des deux flux, vaporisé, comprimé et chauffé dans l'échangeur de type monophasique gaz E2 est ensuite introduite dans un four H1 via la conduite (8) de manière à l'amener à une température d'entrée dans le premier réacteur adiabatique R1 compatible avec la température de la réaction de déshydratation. L'effluent issu du premier réacteur R1 est envoyé dans un deuxième four H2 via la conduite (8 bis) avant d'être introduit dans le deuxième réacteur R2 via la conduite (9 bis).

L'effluent issu du deuxième réacteur R2 subit ensuite les deux échanges successifs décrits précédemment dans les échangeurs E2 et E1 via les conduites (10) et (11).

L'effluent issu de l'échangeur E1 est envoyé via la conduite (12) dans une colonne de séparation gaz/liquide (13) où il est séparé en un effluent comprenant de l'éthylène (14) et un effluent comprenant de l'eau (15). Une partie de l'effluent comprenant de l'eau est recyclé après refroidissement dans la colonne (13) via la conduite (22).

La partie de l'effluent comprenant de l'eau non recyclé dans la colonne (13) est envoyée via la conduite (15) dans une étape (16) de purification et de séparation. Au moins un flux d'eau purifiée (18) et (19) et au moins un flux d'éthanol non converti (17) et (21) sont ensuite séparés. Un flux contenant les gaz légers (20), est également séparé.

Une partie dudit flux d'éthanol non réagi issu de l'étape (16) de purification de l'effluent comprenant de l'eau est recyclé via la conduite (17) et est mélangé avec au moins une partie du flux d'eau purifié recyclé via la conduite (18) dans la conduite (4). Le mélange de ces deux flux est mélangé en amont de l'échangeur E1, à la charge éthanol prétraitée (3).

Les exemples suivants illustrent l'invention sans en limiter la portée.

### Exemples

### Exemple 1 : conforme à l'invention.

L'exemple 1 illustre un procédé selon l'invention dans lequel l'étape c) est mise en oeuvre dans un réacteur adiabatique.

La charge éthanol considérée est produite par fermentation de blé, sans extraction des gluten, par un procédé de type dry milling selon le terme anglo-saxon.

La charge éthanol dont la composition est donnée dans la colonne 1 du tableau 1 est prétraitée sur une résine TA 801 à une température de 140°C. Les caractéristiques de la charge éthanol prétraitée sont également données dans la colonne 2 du tableau 1.

### Étape a)

Ladite charge éthanol prétraitée est introduite, à un débit de 46 187 kg/h, en mélange avec 114 547 kg/h d'eau purifiée recyclée issu de l'étape e) et avec 132 kg/h d'éthanol non converti issu de l'étape e), dans un échangeur E1 à une pression égale à 0,31 MPa.

Le flux d'eau purifié issu de l'étape e) joue le rôle de diluant réactionnel thermique. La dilution de ladite charge éthanol par ajout d'une partie du flux d'eau purifié issu de l'étape e) est réalisée dans un rapport massique diluant sur charge égal à 2,5.

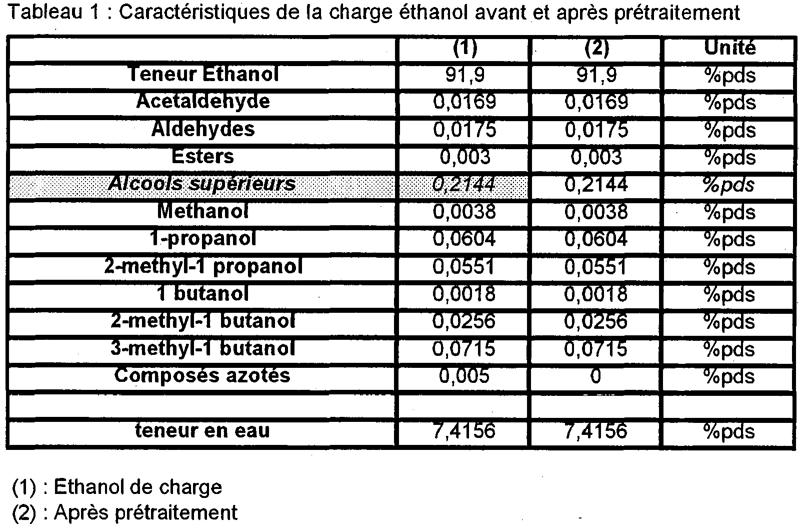

Par souci de simplification, la description des impuretés dans la charge prétraitée a été ôtée dans la suite du texte.

Dans l'étape a), la majorité de la chaleur latente de la phase aqueuse de l'effluent issu du réacteur adiabatique de l'étape c) est récupérée pour vaporiser le mélange de la charge et des deux autres flux, sans apport de chaleur externe. Ainsi, 90.1 % de l'eau contenue dans ledit effluent issu du réacteur adiabatique de l'étape c) se trouve sous forme aqueuse liquide. Ainsi, 88.5 MW sont échangés entre le mélange de la charge et des deux autres flux et l'effluent du réacteur.

La température de début de vaporisation de ladite charge est égale à 126°C (à 0,27 MPa) et la température de condensation finale dudit effluent issu du réacteur adiabatique est-l'effluent est de 117°C (à 0,41 MPa).

### Étape b)

Le mélange de la charge et des deux autres flux, vaporisé, issue de l'échangeur est ensuite comprimée dans un compresseur radial à multiplicateur intégré de manière à ce que la pression dudit mélange de la charge et des deux autres flux, vaporisé à l'issue de la compression soit égale à 0,63 MPa.

Le mélange de la charge et des deux autres flux, vaporisé et comprimé est ensuite chauffé dans un échangeur E2 de type monophasique gaz, grâce à un échange de chaleur avec l'effluent issu du réacteur adiabatique de l'étape c). Dans ledit échangeur de type monophasique gaz, ledit mélange de la charge et des deux autres flux, vaporisé et comprimé est surchauffé à une température de 345°C et l'effluent issu, à l'état gazeux, du réacteur adiabatique de l'étape c) est "désurchauffé" sans être condensé et présente une température de 269°C.

### Étape c)

Ledit mélange de la charge et des deux autres flux, vaporisé, comprimé et chauffé dans ledit échangeur de type monophasique gaz est ensuite introduit dans un four de manière à l'amener à une température d'entrée dans ledit réacteur adiabatique compatible avec la température de la réaction de déshydratation, c'est à dire à une température de 500°C.

Ledit mélange de la charge et des deux autres flux, vaporisé, comprimé et chauffé est introduit dans le réacteur adiabatique à une pression d'entrée de 0,53 MPa.

Le réacteur adiabatique contient un lit fixe de catalyseur de déshydratation, ledit catalyseur comprenant 80% poids de zéolithe ZSM-5 traitée avec H₃PO₄ de manière à ce que la teneur en P₂O₅ soit de 3,5% poids.

Les conditions de température et de pression des flux entrant et sortant du réacteur adiabatique de l'étape c) sont données dans le tableau 2 :

**Tableau 2 : Conditions opératoires de l'étape c) de déshydratation.**

| | **Unité** | **Entrée** | **Sortie** |
|---|---|---|---|
| Pression | MPa | 0,53 | 0,50 |
| Vitesse pondérale horaire | h⁻¹ | 7 | 7 |
| Température de réaction | °C | 500 | 384 |

La conversion de la charge éthanol dans l'étape c) est de 99,4%.

### Étape d)

L'effluent issu du réacteur adiabatique de l'étape c) subit ensuite les deux échanges de chaleur précédemment décrits et est envoyé dans une colonne de séparation gaz/liquide. Un effluent comprenant de l'éthylène à une pression égale à 0,39 MPa est séparé ainsi qu'un effluent comprenant de l'eau. Cette séparation est réalisée par l'utilisation d'une colonne de séparation gas/liquide, avec recyclage de l'eau produite en fond de colonne vers la tête de colonne et après refroidissement et injection d'agent neutralisant.

L'effluent comprenant de l'éthylène subit ensuite une compression pour remonter sa pression à 2,78 MPa avant sa purification finale. L'éthylène séparé n'est pas recyclé dans ledit réacteur adiabatique.

### Étape e)

Un flux d'eau purifiée et un flux d'éthanol non converti ainsi qu'un flux contenant les gaz légers sont ensuite séparés par distillation classique à basse pression de l'eau brute.

### Étape f)

Une partie du flux d'eau purifiée et une partie du flux d'éthanol non converti sont recyclés en amont à l'étape a) de vaporisation dans les proportions décrites dans l'étape a).

Les différents flux, en kg/h, sont renseignés dans les tableaux 3 et 4 :

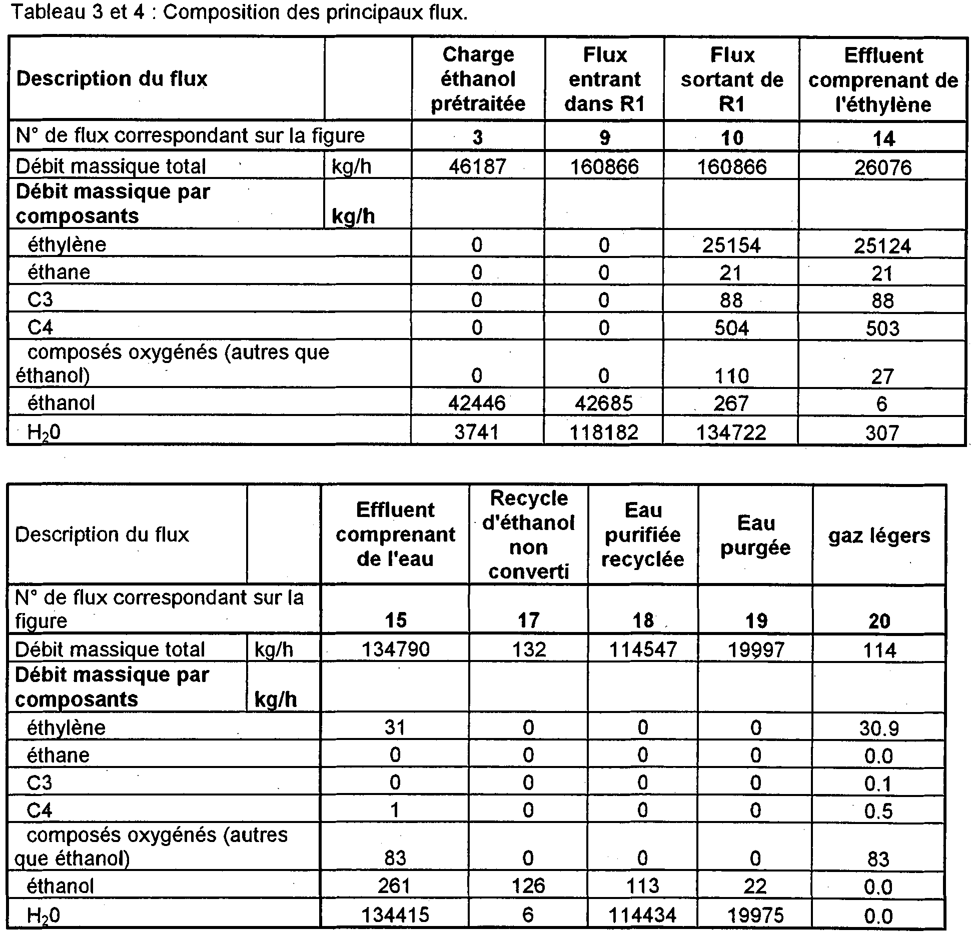

Les composés C3 et C4 sont des composés hydrocarbonés en C3 et C4.

La sélectivité du procédé en éthylène est de 97 % .

Elle est calculée de la façon suivante : (Éthylène contenu dans l'effluent comprenant de l'éthylène) / (0.61 *quantité d'éthanol converti) où la quantité d'éthanol converti est l'éthanol contenu dans la charge éthanol prétraitée soustrait de l'éthanol contenu dans les flux d'eau purgée et dans l'effluent comprenant de l'éthylène. 0,61 g est la quantité maximale d'éthylène obtenue en déshydratant 1 g d'éthanol pur.

Le bilan énergétique du schéma selon l'exemple 1 conforme à l'invention est renseigné dans le tableau 5 :

**Tableau 5 : bilan énergétique**

| **Énergie échangée à l'intérieur du système** | | **Énergie fournie au système par un apport externe** | | |
|---|---|---|---|---|
| Quantité de chaleur échangée dans le premier échangeur(E1) | Quantité de chaleur échangée dans le second échangeur(E2) | Quantité de chaleur échangée dans le four | Électricité requise pour la compression | Quantité de chaleur extraite sur la colonne de séparation gaz/ liquide |
| MW | MW | MW | MW | MW |
| 88.5 | 10.8 | 15.4 | 8.4 | 13.8 |

L'estimation de la consommation en énergie primaire a été réalisée en utilisant les bases suivantes :
- efficacité de 0.8 sur les fours
- efficacité de 0.375 sur la production d'électricité.

Le schéma selon l'exemple 1 conforme à l'invention présente une consommation en énergie primaire équivalente ou consommation spécifique de 6 GJ équivalent par tonne d'éthylène produit.

### Exemple 2 : conforme à l'invention.

L'exemple 2 illustre un procédé selon l'invention dans lequel l'étape c) est mise en oeuvre dans deux réacteurs adiabatiques.

### Étape a)

La même charge éthanol prétraitée que celle utilisée dans l'exemple 1 est introduite avec un débit de 46187 kg/h dans un échangeur E1 à une pression égale à 0.31 MPa, en mélange avec 114549 kg/h d'eau purifiée recyclée et avec 131 kg/h d'éthanol non converti, issus de l'étape e).

### Étape b)

L'échange de chaleur décrit dans l'exemple 1 a lieu et le mélange de la charge et des deux flux, vaporisé, est ensuite comprimé dans un compresseur de même type que celui de l'exemple 1 de manière à ce que la pression dudit mélange de la charge et des deux flux, vaporisé à l'issue de la compression soit égale à 0.69 MPa. 90.2 % de l'eau contenue dans l'effluent issu du dernier réacteur se trouve sous forme aqueuse liquide. Ainsi, 88.9 MW sont échangés entre mélange de la charge et des deux flux et l'effluent issu du dernier réacteur.

La température de début de vaporisation de ladite charge est égale à 126°C (à 0,27 MPa) et la température de condensation finale dudit effluent issu du réacteur adiabatique est-l'effluent est de 117°C (à 0,41 MPa).

### Étape c)

Le mélange de la charge et des deux flux, vaporisé et comprimé est ensuite chauffé dans un échangeur E2 de type monophasique gaz, grâce à un échange de chaleur avec l'effluent issu du deuxième réacteur adiabatique de l'étape c). Dans ledit échangeur de type monophasique gaz, le mélange de la charge et des deux flux, vaporisé et comprimé est surchauffé à une température de 353°C et l'effluent issu, à l'état gazeux, du réacteur adiabatique de l'étape c) est "désurchauffé" sans être condensé et présente une température de 275°C.

Le mélange de la charge et des deux flux, vaporisé, comprimé et chauffé dans ledit échangeur de type monophasique gaz est ensuite introduite dans un four de manière à l'amener à une température d'entrée dans le premier réacteur adiabatique compatible avec la température de la réaction de déshydratation, c'est à dire à une température de 400°C.

Le mélange de la charge et des deux flux, vaporisé, comprimé et chauffé est introduite dans le premier réacteur adiabatique à une pression d'entrée de 0,62 MPa.

L'effluent issu du premier réacteur adiabatique sort dudit premier réacteur à une température de 318°C et est ensuite introduit dans un four de manière à ce que la température d'entrée dudit effluent dans le deuxième réacteur adiabatique soit de 405°C. Ledit effluent présente une pression d'entrée dans ledit deuxième réacteur de 0,53 MPa.

L'effluent issu du deuxième réacteur adiabatique sort dudit deuxième réacteur adiabatique à une température de 380°C et à une pression de 0,50 MPa.

Les deux réacteurs adiabatiques contiennent chacun un lit fixe de catalyseur de déshydratation, ledit catalyseur étant identique dans les deux réacteurs et identique à celui utilisé dans l'exemple 1.

Les conditions de température et de pression des flux entrant et sortant des réacteurs adiabatiques de l'étape c) sont données dans le tableau 6 :

**Tableau 6 : Conditions opératoires de l'étape c) de déshydratation.**

| | Unité | **réacteur 1** | | **réacteur 2** | |
|---|---|---|---|---|---|
| | | entrée | sortie | entrée | sortie |
| Pression | MPa | 0,59 | 0,56 | 0,53 | 0,50 |
| Vitesse pondérale horaire | h⁻¹ | 14 | | 14 | |
| Temperature de reaction | °C | 400 | 318 | 405 | 380 |

La conversion de la charge éthanol à l'issue de l'étape c) est de 99.4%.

### Étape d)

L'effluent issu du deuxième réacteur adiabatique de l'étape c) subit ensuite les deux échanges de chaleur précédemment décrits et est envoyé dans une colonne de séparation gaz/liquide. Un effluent comprenant de l'éthylène à une pression égale à 0,39 MPa est séparé ainsi qu'un effluent comprenant de l'eau. Cette séparation est réalisée par l'utilisation d'une colonne de séparation gaz/liquide, avec recyclage de l'eau produite en fond de colonne vers la tête de colonne après refroidissement et injection d'agent neutralisant

L'effluent comprenant de l'éthylène subit ensuite avantageusement une compression pour remonter sa pression à 2,78 MPa avant sa purification finale. L'éthylène séparé n'est pas recyclé dans le premier ou le deuxième réacteur adiabatique.

### Étape e)

Un flux d'eau purifiée et un flux d'éthanol non converti ainsi qu'un flux contenant les gaz légers sont ensuite séparés par distillation classique à basse pression de l'eau brute.

### Étape f)

Une partie du flux d'eau purifié et une partie du flux d'éthanol non converti sont recyclés en amont de l'étape a) de vaporisation dans les proportions décrites dans l'étape a).

Les différents flux, en kg/h, sont renseignés dans les tableaux 7 et 8 :

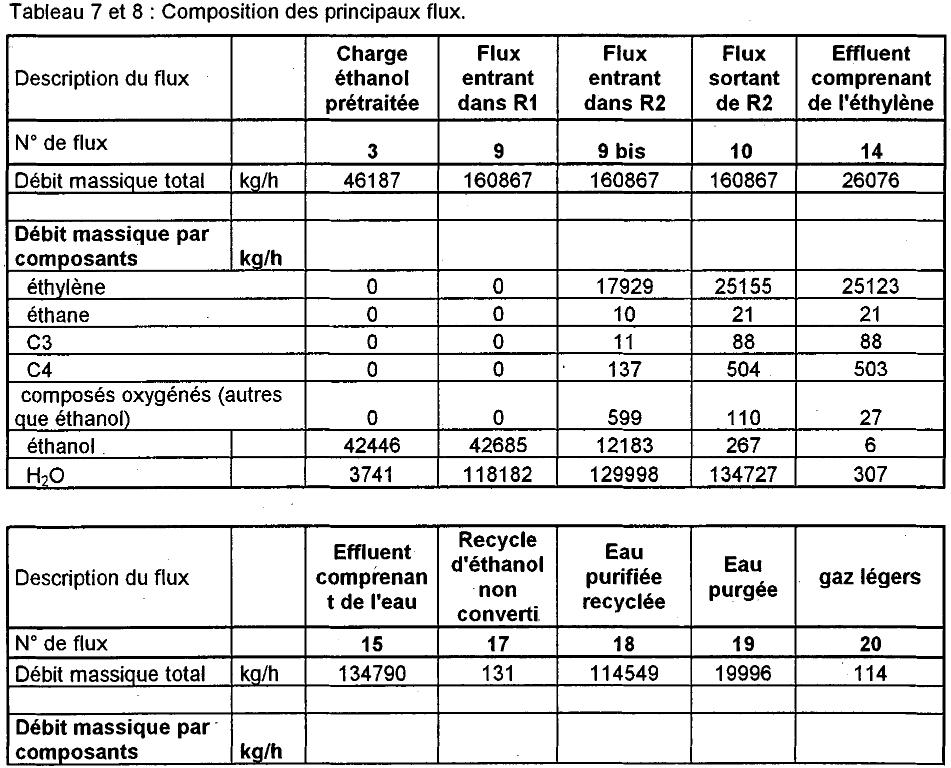

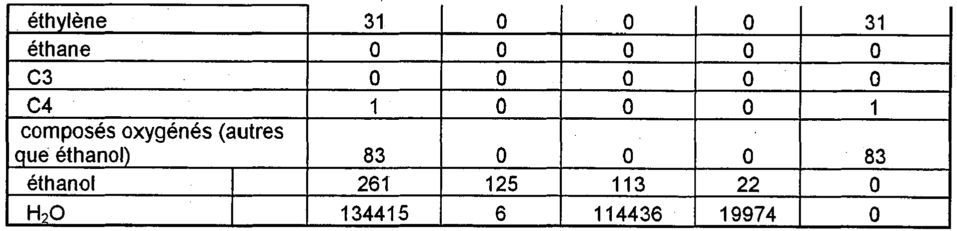

La sélectivité du procédé en éthylène est de 97% . Elle est calculée de la même façon que pour l'exemple 1.

Le bilan énergétique du schéma selon l'exemple 1 conforme à l'invention est renseigné dans le tableau 9 :

**Tableau 9 : bilan énergétique**

| **Énergie échangée à l'intérieur du système** | | **Énergie fournie au système par un apport externe** | | | |
|---|---|---|---|---|---|
| Quantité de chaleur échangée dans le 1^{er} échangeur E1 | Quantité de chaleur échangée dans le 2^{nd} échangeur E2 | Quantité de chaleur échangée dans le 1er four | Quantité de chaleur échangée dans le 2^{nd} four | Électricité requise pour la compression | Quantité de chaleur extraite sur la colonne de séparation gaz/liquide |
| MW | MW | MW | MW | MW | MW |
| 88.9 | 9.9 | 4.6 | 8.4 | 9.5 | 14.0 |

L'estimation de la consommation en énergie primaire a été réalisée en utilisant les mêmes bases que pour le schéma 1.

Le schéma selon l'exemple 2 conforme à l'invention présente une consommation en énergie primaire équivalente ou consommation spécifique de 6 GJ équivalent par tonne d'éthylène produit.

### Exemple 3 : comparatif

L'exemple 3 illustre un procédé dans lequel la réaction de déshydratation est mise en oeuvre dans un réacteur adiabatique et dans lequel la charge, en mélange avec un flux d'éthanol non converti et avec un flux d'eau purifié, est introduite à basse pression dans l'étape a) de vaporisation et ledit mélange, vaporisé, en sortie de l'échangeur ne subit pas d'étape b) de compression. Dans cet exemple, l'éthylène séparé n'est pas recyclé dans ledit réacteur adiabatique contenant le catalyseur de déshydratation.

La même charge éthanol prétraitée que celle utilisée dans l'exemple 1 est introduite, avec un débit de 46 066 kg/h dans un échangeur à une pression égale à 0,65 MPa, en mélange avec 114 553 kg/h d'eau purifiée recyclée et avec 131 kg/h d'éthanol non converti issu de l'étape e). Le mélange de la charge éthanol avec les deux autres flux décrits ci dessus est partiellement vaporisé par échange de chaleur entre ledit mélange et l'effluent issu du réacteur adiabatique. Seule une partie de la chaleur latente de condensation de la phase aqueuse de l'effluent peut être utilisée pour vaporiser en partie ledit mélange de la charge éthanol avec les deux autres flux. Ainsi, seul 33,3% poids dudit mélange est vaporisé et seuls 12% de l'effluent aqueux se trouve condensé, ce qui correspond à une quantité de chaleur échangée de 31,8 MW. Afin de vaporiser en totalité ledit mélange, une quantité de chaleur supplémentaire de 58,2 MW doit être apportée par une source de chaleur extérieure : ledit mélange partiellement vaporisé est ensuite vaporisé totalement dans un échangeur de type évaporateur, utilisant la vapeur comme fluide caloporteur.

Ledit mélange partiellement vaporisé, puis évaporé dans ledit échangeur de type évaporateur est ensuite introduit dans un four de manière à l'amener à une température d'entrée dans ledit réacteur adiabatique compatible avec la température de la réaction de déshydratation, c'est à dire à une température de 500°C.

Ladite charge vaporisée et chauffée est introduite dans le réacteur adiabatique à une pression d'entrée de 0,53 MPa.

Le réacteur adiabatique contient un lit fixe de catalyseur de déshydratation, ledit catalyseur étant identique à celui utilisé dans l'exemple 1.

Les conditions de température et de pression dans ledit réacteur adiabatique sont les suivantes :

**Tableau 10 : Conditions opératoires de l'étape c) de déshydratation.**

| | **Unité** | **Entrée** | **Sortie** |
|---|---|---|---|
| Pression | MPa | 0,53 | 0,50 |
| Vitesse pondérale horaire | h⁻¹ | 7 | 7 |
| Température de reaction | °C | 500 | 383 |

La conversion de la charge éthanol est de 99,4%.

L'effluent issu du réacteur adiabatique de l'étape c) subit ensuite l'échange de chaleur précédemment décrit : il est refroidi jusqu'à 144°C et devra être refroidi dans un échangeur utilisant un fluide frigoporteur extérieur pour atteindre 117°C avant d'être envoyé dans une colonne de séparation gaz/liquide. Cet échangeur est un refroidisseur fonctionnant à l'eau. Une quantité de chaleur de 68 MW devra être ainsi échangée entre l'effluent du réacteur et le fluide frigoporteur. Un effluent comprenant de l'éthylène à une pression égale à 0,38 MPa est séparé ainsi qu'un effluent comprenant de l'eau. Cette séparation est réalisée par l'utilisation d'une colonne de séparation gaz/liquide, avec recyclage de l'eau produite en fond de colonne vers la tête de colonne après refroidissement et injection d'agent neutralisant.

L'effluent comprenant de l'éthylène subit ensuite avantageusement une compression pour remonter sa pression à 2,78 MPa avant sa purification finale. L'éthylène séparé n'est pas recyclé dans ledit réacteur adiabatique.

Un flux d'eau purifiée et un flux d'éthanol non converti ainsi qu'un flux contenant les gaz légers sont ensuite séparés par distillation classique à basse pression de l'eau brute.

Un flux d'eau purifiée et un flux d'éthanol non converti sont ensuite séparée par distillation classique à basse pression de l'eau brute.

Une partie du flux d'eau purifié et une partie du flux d'éthanol non converti sont recyclés en amont de l'étape a) de vaporisation.

Les différents flux, en kg/h, sont renseignés dans les tableaux 11 et 12 :

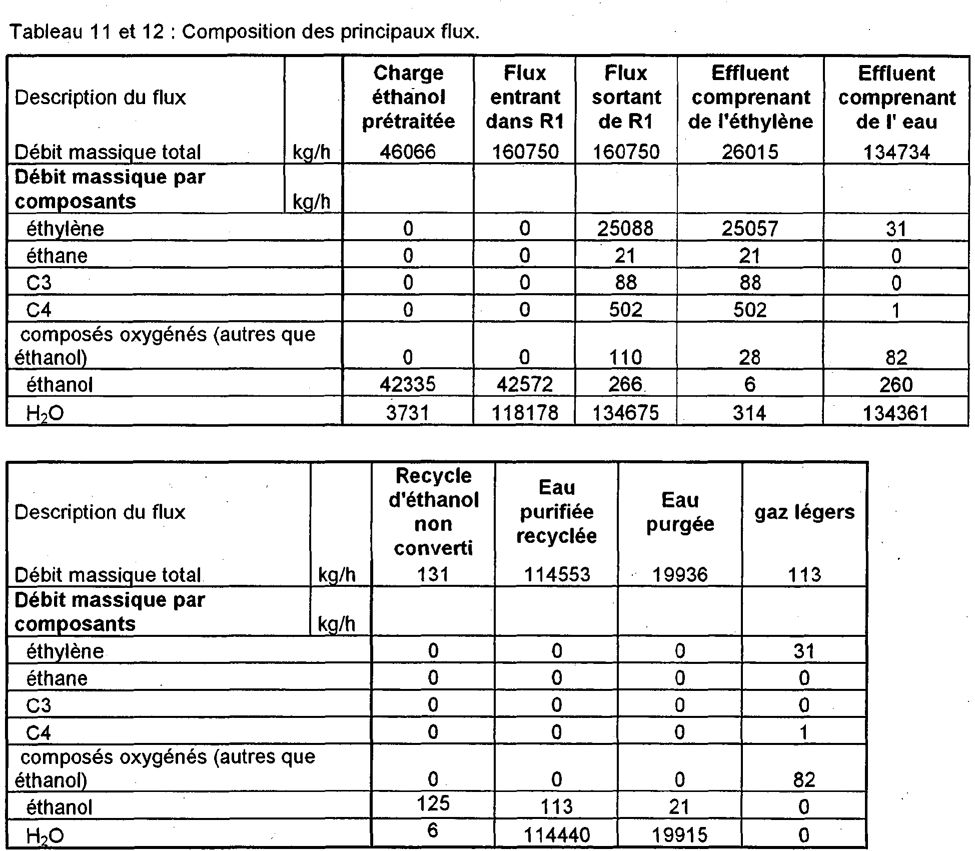

La sélectivité du procédé en éthylène est de 97% . Elle est calculée de la même façon que pour l'exemple 1.

Le bilan énergétique du schéma selon l'exemple 3 non conforme à l'invention est renseigné dans le tableau 13 :

**Tableau 13 : bilan énergétique**

| **Énergie échangée à l'intérieur du système** | **Énergie fournie au système par un apport externe** | | | |
|---|---|---|---|---|
| Quantité de chaleur échangée sur 1^{er} échangeur | Quantité de chaleur échangée sur l'évaporateur | Quantité de chaleur échangée sur le four | Quantité de chaleur extraite sur le refroidisseur | Quantité de chaleur extraite sur la colonne de séparation gaz/liquide |
| MW | MW | MW | MW | MW |
| 31.9 | 58.2 | 33.0 | 68.0 | 13.6 |

L'estimation de la consommation en énergie primaire a été réalisée en utilisant les mêmes bases que pour le schéma 1, en considérant en plus, une efficacité de 0.9 sur la production de vapeur.

Ce schéma 3 présente une consommation en énergie primaire équivalente ou consommation spécifique, de 15,2 GJ équivalent par tonne d'éthylène produit. La vaporisation de la charge en mélange avec un flux d'éthanol non converti et un flux d'eau purifié, réalisée dans le schéma 1 de l'exemple 1 selon la présente invention, à basse pression, permet de réduire de façon notable la consommation en énergie primaire équivalente : le schéma 1 présentait une consommation en énergie primaire de 6 GJ équivalent par tonne d'éthylène.

### Exemple 4 : comparatif

L'exemple 4 illustre un procédé dans lequel la réaction de déshydratation est mise en oeuvre dans un réacteur adiabatique et dans lequel la charge, en mélange avec un flux d'éthanol non converti et avec un flux d'eau purifié, est introduite dans l'étape a) de vaporisation et ledit mélange, vaporisé, en sortie de l'échangeur ne subit pas d'étape b) de compression. Dans cet exemple, une partie de l'effluent issu du réacteur adiabatique, comprenant de l'éthylène et de l'eau, est recyclée dans ledit réacteur adiabatique contenant le catalyseur de déshydratation.

L'exemple 4 est basé sur le fait qu'une partie de l'effluent issu du réacteur adiabatique, comprenant de l'éthylène et de l'eau, est comprimé et recyclé en entrée du premier réacteur, ceci dans le but de recycler une partie du fluide caloporteur qui est l'eau directement sous forme vapeur sans condensation et revaporisation. Ce recycle contient cependant de l'éthylène et en conséquence, des réactions secondaires d'oligomérisation, de transfert d'hydrogène et de disproportionation des oléfines vont avoir lieu en quantité plus importantes sur le réacteur, conduisant à une perte globale de production d'éthylène sur le réacteur et donc à une baisse de la sélectivité en éthylène.

La même charge éthanol prétraitée que celle utilisée dans l'exemple 1 est introduite à raison de 46065 kg/h dans un échangeur à une pression égale à 0,65 MPa, en mélange avec 49241 kg/h d'eau purifiée recyclée et avec 205 kg/h d'éthanol non converti. Ledit mélange de la charge éthanol en mélange avec le flux d'eau purifiée et le flux d'éthanol non converti est partiellement vaporisé par échange de chaleur avec l'effluent issu du réacteur adiabatique. Seule une partie de la chaleur latente de condensation de la phase aqueuse de l'effluent peut être utilisée pour vaporiser en partie ledit mélange. Ainsi, seul 42.4% poids dudit mélange est vaporisé et seuls 17 % poids de l'effluent aqueux est condensé, ce qui correspond à une quantité de chaleur échangée de 21,3 MW.

Ledit mélange partiellement vaporisé est ensuite mélangé avec une partie de l'effluent issu du réacteur adiabatique comprenant de l'éthylène et de l'eau, préalablement comprimé, dont le débit est de 20 000 kg/h. L'apport de chaleur lié audit effluent recyclé et comprimé ne suffit pas à vaporiser tout le mélange de la charge éthanol en mélange avec le flux d'eau purifiée et le flux d'éthanol non converti : 88 % poids dudit mélange est vaporisé. Afin de vaporiser totalement ledit mélange, il est nécessaire d'apporter 12,8 MW supplémentaire par une source de chaleur externe : ledit mélange partiellement vaporisé est ensuite vaporisé totalement dans un échangeur de type évaporateur, utilisant de la vapeur comme fluide caloporteur.

Ledit mélange vaporisé, et chauffé dans ledit échangeur de type évaporateur est ensuite introduit dans un four de manière à l'amener à une température d'entrée dans ledit réacteur adiabatique compatible avec la température de la réaction de déshydratation, c'est à dire à une température de 476°C.

Ladite charge vaporisée et chauffée est introduite dans le réacteur adiabatique à une pression d'entrée de 0,53 MPa.

Le réacteur adiabatique contient un lit fixe de catalyseur de déshydratation, ledit catalyseur étant identique à celui utilisé dans l'exemple 1.

Les conditions de température et de pression dans ledit réacteur adiabatique sont les suivantes :

**Tableau 14 : Conditions opératoires.**

| | **Unité** | **Entrée** | **Sortie** |
|---|---|---|---|
| Pression | MPa | 0,53 | 0,50 |
| Vitesse pondérale horaire | h⁻¹ | 7 | 7 |
| Temperature de reaction | °C | 476 | 393 |

La conversion de la charge éthanol est de 98,8%.

L'effluent issu du réacteur adiabatique subit ensuite l'échange de chaleur précédemment décrit est refroidi jusqu' à 117°C par une source extérieure avant d'être envoyé dans une colonne de séparation gaz/liquide. Cet échangeur peut être un refroidisseur fonctionnant à l'eau. Une quantité de chaleur de 30,9 MW devra être ainsi échangée entre l'effluent du réacteur et le fluide frigoporteur. Un effluent comprenant de l'éthylène à une pression égale à 0,43 MPa est séparé ainsi qu'un effluent comprenant de l'eau. Cette séparation est réalisée par l'utilisation d'une colonne de séparation gaz/liquide, avec recyclage de l'eau produite en fond de colonne vers la tête de colonne après refroidissement et injection d'agent neutralisant

L'effluent comprenant de l'éthylène subit ensuite avantageusement une compression pour remonter sa pression à 2,78 MPa avant sa purification finale. L'éthylène séparé n'est pas recyclé dans ledit réacteur adiabatique.

Un flux d'eau purifiée et un flux d'éthanol non converti ainsi qu'un flux contenant les gaz légers sont ensuite séparés par distillation classique à basse pression de l'eau brute.

Une partie du flux d'eau purifié et une partie du flux d'éthanol non converti sont recyclés en amont de l'étape a) de vaporisation.

Les différents flux, en kg/h, sont renseignés dans le tableau 15 :

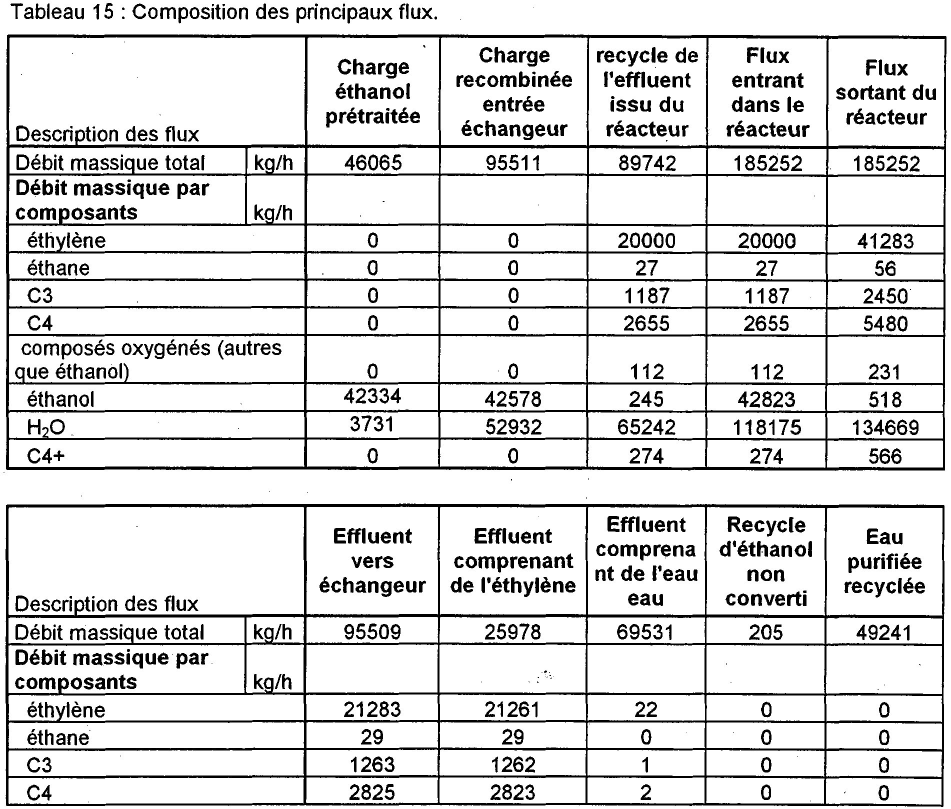

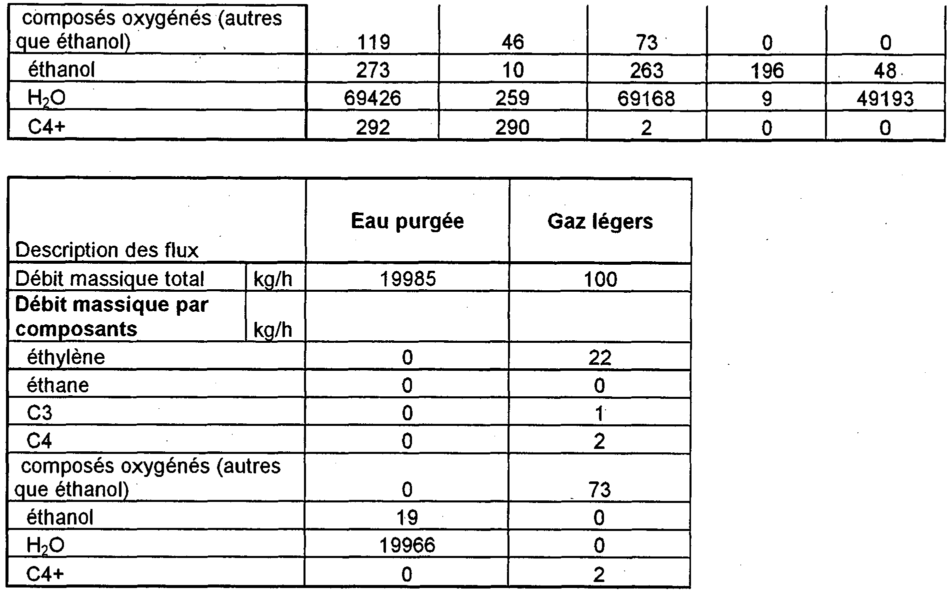

La sélectivité du procédé en éthylène est de 82%. Elle est calculée de la même façon que pour l'exemple 1. On remarque la perte de sélectivité lié au recyclage de l'effluent issu du réacteur adiabatique comprenant de l'éthylène et de l'eau, les précédents schémas ne mettant pas en oeuvre de recyclage dudit effluent comprenant de l'éthylène permettant d'obtenir une sélectivité en éthylène de 97%.

Le bilan énergétique du schéma selon l'exemple 4 non conforme à l'invention est renseigné dans le tableau 16 :

**Tableau 16 : bilan énergétique**

| **Énergie échangée à l'intérieur du système** | **Énergie fournie au système par un apport externe** | | | | |
|---|---|---|---|---|---|
| Quantité de chaleur échangée sur 1^{er} échangeur | Quantité de chaleur échangée sur l'évaporateur | Quantité de chaleur échangée sur le four | Électricité requise pour le compresseur | Quantité de chaleur extraite sur le refroidisseur | Quantité de chaleur extraite sur la colonne de séparation gaz/liquide |
| MW | MW | MW | MW | MW | MW |
| 21,3 | 12,8 | 36,7 | 0,65 | 30,8 | 11,9 |
| source interne | source externe | source externe | source externe | source externe | source externe |

L'estimation de la consommation en énergie primaire a été réalisée en utilisant les mêmes bases que pour le schéma 1, en considérant en plus, une efficacité de 0,9 sur la production de vapeur.

Ce schéma 4 présente une consommation en énergie primaire équivalente ou consommation spécifique de 10,5 GJ équivalent par tonne d'éthylène produit. La vaporisation de la charge en mélange avec un flux d'éthanol non converti et un flux d'eau purifié, réalisée dans le schéma 1 de l'exemple 1 selon la présente invention, à basse pression, permet de réduire de façon notable la consommation en énergie primaire équivalente : le schéma 1 présentait une consommation en énergie primaire de 6 GJ équivalent par tonne d'éthylène.

## Revendications

1. Procédé de déshydratation d'une charge éthanol en éthylène comprenant :
a) la vaporisation de ladite charge éthanol en mélange avec au moins une partie du flux d'eau purifié recyclé selon l'étape f) dans un échangeur grâce à un échange de chaleur avec l'effluent issu du dernier réacteur, ladite charge éthanol en mélange avec au moins une partie dudit flux d'eau purifié recyclé étant introduite dans ladite étape de vaporisation à une pression comprise entre 0,1 et 0,4 MPa,
b) la compression de ladite charge éthanol en mélange avec au moins une partie du flux d'eau purifié recyclé selon l'étape f), vaporisée, dans un compresseur,
c) l'introduction de ladite charge éthanol en mélange avec au moins une partie du flux d'eau purifié recyclé selon l'étape f), vaporisée et comprimée, à une température d'entrée comprise entre 350 et 500°C et à une pression d'entrée comprise entre 0,2 et 1,3 MPa, dans au moins un réacteur adiabatique contenant au moins un catalyseur de déshydratation et dans lequel la réaction de déshydratation a lieu,
d) la séparation de l'effluent issu du dernier réacteur adiabatique de l'étape c) en un effluent comprenant de l'éthylène à une pression inférieure à 1 MPa et un effluent comprenant de l'eau,
e) la purification d'au moins une partie de l'effluent comprenant de l'eau issu de l'étape d) et la séparation d'au moins un flux d'eau purifiée et d'au moins un flux d'éthanol non converti,
f) le recyclage d'au moins une partie du flux d'eau purifiée issu de l'étape e) en amont de l'étape a).

2. Procédé selon la revendication 1 dans lequel est ladite charge éthanol est une charge éthanol produite à partir de source renouvelable issue de la biomasse

3. Procédé selon l'une des revendications 1 ou 2 dans lequel ladite charge éthanol subit une étape de prétraitement préalablement à l'étape a) de vaporisation.

4. Procédé selon l'une des revendications 1 à 3 dans lequel au moins un flux d'éthanol non réagi issu de l'étape e) de purification de l'effluent comprenant de l'eau est également introduit dans l'échangeur de l'étape a) de vaporisation.

5. Procédé selon l'une des revendications 1 à 4 dans lequel ladite charge éthanol en mélange avec au moins une partie du flux d'eau purifié recyclé selon l'étape f) est introduite dans ladite étape a) de vaporisation à une pression inférieure à la pression de l'effluent en sortie du dernier réacteur.

6. Procédé selon l'une des revendications 1 à 5 dans lequel la pression de la charge éthanol en mélange avec au moins une partie du flux d'eau purifié recyclé selon l'étape f), vaporisée à l'issue de l'étape b) de compression, est comprise entre 0,2 et 1,3 MPa.

7. Procédé selon l'une des revendications 1 à 6 dans lequel ladite charge éthanol en mélange avec au moins une partie du flux d'eau purifié recyclé selon l'étape f), vaporisée et comprimée issue de l'étape b) de compression, est chauffée dans un échangeur de type monophasique gaz, grâce à un échange de chaleur avec l'effluent issu du dernier réacteur adiabatique de l'étape c).

8. Procédé selon l'une des revendications 1 à 7 dans lequel l'effluent issu du dernier réacteur adiabatique de l'étape c) présente en sortie du dernier réacteur adiabatique de l'étape c) une température comprise entre 270 et 420°C.

9. Procédé selon l'une des revendications 1 à 8 dans lequel l'effluent issu du dernier réacteur adiabatique de l'étape c) présente en sortie du dernier réacteur adiabatique de l'étape c) une pression comprise entre 0,1 et 1,1 MPa.

10. Procédé selon l'une des revendications 1 à 9 dans lequel l'effluent issu du dernier réacteur adiabatique de l'étape c) n'est pas recyclé en amont de l'étape c), dans au moins un réacteur adiabatique.

11. Procédé selon l'une des revendications 1 à 10 dans lequel l'étape c) dans laquelle la réaction de déshydratation a lieu est réalisée dans un ou deux réacteurs.

12. Procédé selon l'une des revendications 1 à 11 dans lequel ledit catalyseur de déshydratation utilisé dans l'étape c) est un catalyseur acide amorphe ou un catalyseur acide zéolithique.

13. Procédé selon l'une des revendications 1 à 12 dans lequel au moins une partie dudit flux d'éthanol non réagi issu de l'étape e) de purification de l'effluent comprenant de l'eau est recyclé et mélangé à la charge éthanol en amont de l'étape a) de vaporisation de ladite charge.

14. Procédé selon l'une des revendications 1 à 13 dans lequel ladite charge éthanol est une charge éthanol hydratée concentrée.

15. Procédé selon la revendication 14 dans lequel ladite charge éthanol concentrée comprend un pourcentage massique en éthanol compris entre 35 et 99,9% poids.

16. Procédé selon la revendication 15 dans lequel ladite charge éthanol concentrée comprend un pourcentage massique en éthanol compris entre 35 et 96% poids.

## Patentansprüche

1. Verfahren zur Dehydrierung einer Ethanolbeschickung zu Ethylen, umfassend:
a) das Verdampfen der Ethanolbeschickung im Gemisch mit mindestens einem Teil des gereinigten Wasserstroms, der gemäß Schritt f) zurückgeführt wurde, in einem Austauscher mittels eines Wärmeaustausches mit dem Abfluss, der aus dem letzten Reaktor stammt, wobei die Ethanolbeschickung im Gemisch mit mindestens einem Teil des zurückgeführten, gereinigten Wasserstroms in den Schritt zum Verdampfen mit einem Druck im Bereich zwischen 0,1 und 0,4 MPa eingeführt wird,
b) Verdichten der verdampften Ethanolbeschickung im Gemisch mit mindestens einem Teil des gereinigten Wasserstroms, der gemäß Schritt f) zurückgeführt wurde, in einem Verdichter,
c) Einführen der verdampften und verdichteten Ethanolbeschickung im Gemisch mit mindestens einem Teil des gereinigten Wasserstroms, der gemäß Schritt f) zurückgeführt wurde, bei einer Eintrittstemperatur im Bereich zwischen 350 und 500 °C und mit einem Eintrittsdruck im Bereich zwischen 0,2 und 1,3 MPa, in mindestens einen adiabatischen Reaktor, der mindestens einen Dehydrierungskatalysator enthält und in dem die Dehydrierungsreaktion stattfindet,
d) Trennen des Abflusses, der aus dem letzten adiabatischen Reaktor aus Schritt c) stammt, in einen Abfluss, der Ethylen bei einem Druck kleiner 1 MPa umfasst, und einen Abfluss, der Wasser umfasst,
e) Reinigen mindestens eines Teils des Abflusses, der Wasser, das aus Schritt d) stammt, umfasst, und Trennen des mindestens einen Stroms aus gereinigten Wasser und des mindestens einen nicht umgewandelten Ethanolstroms,
f) Rückführen des mindestens einen Teils des gereinigten Wasserstroms, der aus Schritt e) stammt, stromaufwärts von Schritt a).

2. Verfahren nach Anspruch 1, wobei die Ethanolbeschickung eine Ethanolbeschickung ist, die aus einer aus Biomasse stammenden erneuerbaren Quelle erzeugt wird.

3. Verfahren nach einem der Ansprüche 1 oder 2, wobei die Ethanolbeschickung vor dem Schritt a) zum Verdampfen einem Schritt zur Vorbehandlung unterzogen wird.

4. Verfahren nach einem der Ansprüche 1 bis 3, wobei mindestens ein nicht umgesetzter Ethanolstrom, der aus Schritt e) zur Reinigung des Abflusses stammt, der Wasser umfasst, ebenfalls in den Austauscher von Schritt a) zum Verdampfen eingeführt wird.

5. Verfahren nach einem der Ansprüche 1 bis 4, wobei die Ethanolbeschickung im Gemisch mit mindestens einem Teil des gereinigten Wasserstroms, der gemäß Schritt f) zurückgeführt wurde, in den Schritt a) zum Verdampfen mit einem Druck eingeführt wird, der kleiner ist als der Druck des Abflusses am Ausgang des letzten Reaktors.

6. Verfahren nach einem der Ansprüche 1 bis 5, wobei der Druck der Ethanolbeschickung im Gemisch mit mindestens einem Teil des gereinigten Wasserstroms, der gemäß Schritt f) zurückgeführt wurde, die am Ausgang von Schritt b) zum Verdichten verdampft wurde, im Bereich zwischen 0,2 und 1,3 MPa liegt.

7. Verfahren nach einem der Ansprüche 1 bis 6, wobei die Ethanolbeschickung im Gemisch mit mindestens Teil des gereinigten Wasserstroms, der gemäß Schritt f) zurückgeführt wurde, die am Ausgang von Schritt b) zum Verdichten verdampft und verdichtet wurde, in einem einphasigen Gas-Austauscher mithilfe eines Wärmeaustausches mit dem Abfluss, der aus dem letzten adiabatischen Reaktor aus Schritt c) stammt, erhitzt wird.

8. Verfahren nach einem der Ansprüche 1 bis 7, wobei der Abfluss, der aus dem letzten adiabatischen Reaktor von Schritt c) stammt, am Ausgang des letzten adiabatischen Reaktors von Schritt c) eine Temperatur im Bereich zwischen 270 und 420 °C aufweist.

9. Verfahren nach einem der Ansprüche 1 bis 8, wobei der Abfluss, der aus dem letzten adiabatischen Reaktor von Schritt c) stammt, am Ausgang des letzten adiabatischen Reaktors von Schritt c) einen Druck im Bereich zwischen 0,1 und 1,1 MPa aufweist.

10. Verfahren nach einem der Ansprüche 1 bis 9, wobei der Abfluss, der aus dem letzten adiabatischen Reaktor von Schritt c) stammt, stromaufwärts von Schritt c) nicht in mindestens einen adiabatischen Reaktor zurückgeführt wird.

11. Verfahren nach einem der Ansprüche 1 bis 10, wobei der Schritt c), in dem die Dehydrierungsreaktion stattfindet, in einem oder zwei Reaktoren durchgeführt wird.

12. Verfahren nach einem der Ansprüche 1 bis 11, wobei der in Schritt c) verwendete Dehydrierungskatalysator ein amorpher Säurekatalysator oder ein zeolithischer Säurekatalysator ist.

13. Verfahren nach einem der Ansprüche 1 bis 12, wobei mindestens ein Teil des nicht umgesetzten Ethanolstroms, der aus Schritt e) zur Reinigung des Abflusses stammt, der Wasser umfasst, nicht zurückgeführt und mit der Ethanolbeschickung stromaufwärts von Schritt a) zum Verdampfen der Beschickung gemischt wird.

14. Verfahren nach einem der Ansprüche 1 bis 13, wobei es sich bei der Ethanolbeschickung um eine konzentrierte, hydratisierte Ethanolbeschickung handelt.

15. Verfahren nach Anspruch 14, wobei die konzentrierte Ethanolbeschickung einen Masseprozentsatz an Ethanol im Bereich zwischen 35 und 99,9 Gew.-% umfasst.

16. Verfahren nach Anspruch 15, wobei die konzentrierte Ethanolbeschickung einen Masseprozentsatz an Ethanol im Bereich zwischen 35 und 96 Gew.-% umfasst.

## Claims

1. Process for dehydration of an ethanol feedstock into ethylene comprising:
a) The vaporization of said ethanol feedstock in a mixture with at least a portion of the purified water stream that is recycled according to stage f) in an exchanger owing to a heat exchange with the effluent that is obtained from the last reactor, with said ethanol feedstock in a mixture with at least a portion of said purified water stream that is recycled being introduced into said vaporization stage at a pressure of between 0.1 and 0.4 MPa,
b) The compression of said ethanol feedstock in a mixture with at least a portion of the purified water stream that is recycled according to stage f), vaporized, in a compressor,
c) The introduction of said ethanol feedstock that is in a mixture with at least a portion of the purified water flow that is recycled according to stage f), vaporized and compressed, at an entrance temperature of between 350 and 500°C and at an entrance pressure of between 0.2 and 1.3 MPa, in at least one adiabatic reactor that contains at least one dehydration catalyst and in which the dehydration reaction takes place,
d) The separation of the effluent that is obtained from the last adiabatic reactor of stage c) in an effluent that comprises ethylene at a pressure that is lower than 1 MPa and an effluent that comprises water,
e) The purification of at least a portion of the effluent comprising water that is obtained from stage d) and the separation of at least one stream of purified water and at least one unconverted ethanol stream,
f) The recycling of at least a portion of the purified water stream that is obtained from stage e), upstream from stage a).

2. Process according to Claim 1, in which said ethanol feedstock is an ethanol feedstock that is produced from a renewable source that is obtained from biomass.

3. Process according to one of Claims 1 or 2, in which said ethanol feedstock undergoes a pretreatment stage prior to the vaporization stage a).

4. Process according to one of Claims 1 to 3, in which at least one unreacted ethanol stream that is obtained from stage e) for purification of the effluent that comprises water is also introduced into the exchanger of vaporization stage a).

5. Process according to one of Claims 1 to 4, in which said ethanol feedstock that is mixed with at least a portion of the purified water stream that is recycled according to stage f) is introduced into said stage a) for vaporization at a pressure that is lower than the pressure of the effluent at the outlet of the last reactor.

6. Process according to one of Claims 1 to 5, in which the pressure of the ethanol feedstock that is mixed with at least a portion of the purified water stream that is recycled according to stage f), vaporized at the end of compression stage b), is between 0.2 and 1.3 MPa.

7. Process according to one of Claims 1 to 6, in which said ethanol feedstock that is mixed with at least a portion of the purified water stream that is recycled according to stage f), vaporized and compressed, obtained from compression stage b), is heated in a gas single-phase-type exchanger, owing to a heat exchange with the effluent that is obtained from the last adiabatic reactor of stage c).

8. Process according to one of Claims 1 to 7, in which the effluent that is obtained from the last adiabatic reactor of stage c) has a temperature of between 270 and 420°C at the outlet of the last adiabatic reactor of stage c).

9. Process according to one of Claims 1 to 8, in which the effluent that is obtained from the last adiabatic reactor of stage c) has a pressure of between 0.1 and 1.1 MPa at the outlet of the last adiabatic reactor of stage c).

10. Process according to one of Claims 1 to 9, in which the effluent that is obtained from the last adiabatic reactor of stage c) is not recycled upstream from stage c), in at least one adiabatic reactor.

11. Process according to one of Claims 1 to 10, in which stage c), in which the dehydration reaction takes place, is carried out in one or two reactors.

12. Process according to one of Claims 1 to 11, in which said dehydration catalyst that is used in stage c) is an amorphous acid catalyst or a zeolitic acid catalyst.

13. Process according to one of Claims 1 to 12, in which at least a portion of said unreacted ethanol stream that is obtained from stage e) for purification of the effluent that comprises water is recycled and mixed with the ethanol feedstock that is upstream from stage a) for vaporization of said feedstock.

14. Process according to one of Claims 1 to 13, in which said ethanol feedstock is a concentrated hydrated ethanol feedstock.

15. Process according to Claim 14, in which said concentrated ethanol feedstock comprises a percent by mass of ethanol that is between 35 and 99.9% by weight.

16. Process according to Claim 15, in which said concentrated ethanol feedstock comprises a percent by mass of ethanol that is between 35 and 96% by weight.
